Europäisches Patentamt

European Patent Office

Office européen des brevets

(11) Veröffentlichungsnummer: **0 214 435 B1**

# EUROPÄISCHE PATENTSCHRIFT

(45) Veröffentlichungstag der Patentschrift: **29.07.92**

(21) Anmeldenummer: **86110342.2**

(22) Anmeldetag: **26.07.86**

(51) Int. Cl.5: **C12N 15/00**, C12N 1/20, C12N 9/56, C12N 15/57, //(C12N1/20,C12R1:10,1:125)

(54) **Alkalische Protease, Verfahren zur Herstellung von Hybridvektoren und genetisch transformierte Mikroorganismen.**

(30) Priorität: **03.08.85 DE 3527913**

(43) Veröffentlichungstag der Anmeldung:
**18.03.87 Patentblatt 87/12**

(45) Bekanntmachung des Hinweises auf die Patenterteilung:
**29.07.92 Patentblatt 92/31**

(84) Benannte Vertragsstaaten:
**AT BE CH DE FR GB IT LI LU NL SE**

(56) Entgegenhaltungen:
**EP-A- 0 006 638
EP-A- 0 108 301
EP-A- 0 130 756
WO-A-86/01903
DE-A- 2 925 427**

**CHEMICAL ABSTRACTS, Band 104, Nr. 19, Mai 1986, Seite 164, Ref. Nr. 163023q; Columbus, Ohio, US & JP-A-61 12 287 (LION CORP.) 20-01-1986**

(73) Patentinhaber: **Henkel Kommanditgesellschaft auf Aktien
Postfach 1100 Henkelstrasse 67
W-4000 Düsseldorf-Holthausen(DE)**

(72) Erfinder: **Berger, Harald, Dr.
Zur Verlach 63
W-4010 Hilden(DE)**
Erfinder: **Goebel, Werner, Prof. Dr.
Ravensburger Strasse 2 b
W-8707 Veitshöchheim(DE)**
Erfinder: **Kreft, Jürgen, Dr.
Bayern-Strasse 97
W-8700 Höchberg(DE)**
Erfinder: **Bartnik, Friedhelm, Dr.
Melanchthonstrasse 11
W-4000 Düsseldorf(DE)**

CHEMICAL ABSTRACTS, Band 88, 1978, Seite 245, Ref. Nr. 117507d; Columbus, Ohio, US; B.K. SCHREMPF et al.: "Bacteriocin and antibiotic resistance plasmids in Bacillus cereus and Bacillus subtilis" & J. BACTERIOL. 1978, 133 (2), 897-903

Chemical abstracts, Band 103, n 3, Juli 1985, Seite 467, Ref-Nr 21225n, Columbus, Ohio, US & JP a 6030685 (Lion Corp.)

Chemical abstracts, Band 68, 1968, Seite 10773, Ref.Nr 111802m, Columbus, Ohio, US. E.L. Smith et al "Subtilisin Carlsberg V. The complete sequence ; comparison with subtilisin BPN', evolutionary relationships" & Biol. Chem. 243(9), 2184-91

Nucleic acids Research, Band 13, Nr 24, Dezember 1985, Seiten 8913-8926, Oxford GB. M. Jacobs et al : "Cloning, sequencing and expression of subtilisin Carlsberg from Bacillus licheniformis"

Nucleic acids Research, Band 11, Nr 22, 1983, Seiten 7911-7925, Oxford GB. J.A. Wells et al "Cloning, sequencing and secretion of Bacillus amyloliquefaciens subtilisin in Bacillus subtilis"

Chemical abstracts, Band 102, 1985, Seite

136, Ref.Nr 90798, Columbus, Ohio, US. J. Okada et al "Cloning of the gene responsible for the extra-cellular proteolytic activities of Bacillus licheniformis" & Appl. Micorbiol. Biotechnol. 1984, 20(6), 406-12

Proc. Natl. Acad. Sci. USA, Band 75, Nr 6, Juni 1978, Biochemistry, Seiten 2746-2749, US. S. Broome et al : "Immunological screening method to detect specific translation products"

Chemical abstracts, Band 78, Mai 1973, Seite 230, Ref.Nr 134334f, Columbus, Ohio, US. C.E. Stauffer : "Binding of rabbit immunoglobulin G Fab fragments to subtilisin Carlsberg" & Immunochemistry 1973 10(3), 129-37

Chemical abstracts, Band 95, Nr 13, 28 September 1981, Seite 272, Ref. Nr 110757w, Columbus, Ohio, US. K.A. Shaginyan et al "Immunological comparison of intracellular and extracellularserine protease from Bacillus amyloliquefaciens and some other serine proteases" & Biokhimiya (Moscow) 1981 46(7), 1290-7

Gene, Band 16, 1981, Seiten 149-159, Elsevier/North Holland, Biomedical Press. P. Buckel et al "Expression of pseudomonas fluorescens D-galactose dehydrogenase in E. coli"

**Beschreibung**

Die Erfindung betrifft eine Methode zur Transformation von Mikroorganismen der Gattung Bacillus zu Bildnern der Protease Subtilisin Carlsberg und ihrer Varianten. Beschrieben wird die Herstellung doppelsträngiger Desoxyribonukleinsäuren, die nur für Subtilisin Carlsberg oder seine Varianten kodieren, Hybridvektoren, die diese isolierten doppelsträngigen Desoxyribonukleinsäuren zusammen mit dem Strukturgen des Leaderproteins, Start- und Stopcodons in operabler Reihenfolge, Ribosomen-Bindungsstelle und Promotor enthalten, sowie Wirtsorganismen der Gattung Bacillus für diese Hybridplasmide.

Es sind zahlreiche Proteasen bekannt, die von Mikroorganismen, insbesondere Bakterien und Pilzen gebildet werden und durch aktiven Transport aus der Zelle ausgeschleust werden, so daß sie sich im Kulturmedium sammeln. Zahlreiche Enzyme dieser Art finden bereits technische Anwendung in Verfahren, bei denen es darum geht, Proteine abzubauen. Wichtige Anwendungen sind beispielsweise Wasch- und Reinigungsmittel oder auch Tiernahrungsmittel. Die Proteasen werden gängigerweise nach dem pH-Bereich eingeteilt, in dem sie ihr Wirkungsoptimum zeigen. Man spricht daher von alkalischen, neutralen und sauren Proteasen. Die alkalischen Proteasen sind von besonderer Wichtigkeit in Wasch- und Reinigungsmittel. Dabei wird eine wichtige Gruppe unter dem Oberbegriff Subtilisin zusammengefaßt. Diese Bezeichnung leitet sich von Bacillus subtilis ab, da u.a. Stämme dieser Spezies in der Lage sind, Subtilisin zu erzeugen. In den letzten Jahren ist es gelungen, verschiedene in der Natur vorkommende Arten von Subtilisin voneinander zu trennen, und strukturell aufzuklären (E.L. Smith et al, J. Biol. Chem. (1968) 243 (9), 2184. Dabei hat sich gezeigt, daß für die Praxis insbesondere zwei Arten von Subtilisin von Bedeutung sind, nämlich Subtilisin BPN' und Subtilisin Carlsberg. Subtilisin BPN' wird oftmals aus Bacillus amyloliquefaciens gewonnen, wo hingegen sich Subtilisin Carlsberg beispielsweise durch Kultivierung von Stämmen der Spezies Bacillus licheniformis erhalten läßt. Viele Bacillus-Stämme scheiden beide Proteasen oftmals im Gemisch mit neutralen Proteasen (Metallo-Proteasen) aus.

Aufgrund der technischen Bedeutung der Enzyme, die für die Protease Subtilisin Carlsberg etwas höher anzusetzen ist als für die Protease Subtilisin BPN', hat es in der Vergangenheit bereits zahlreiche Versuche gegeben, Stämme aufzufinden, die das eine oder andere Enzym oder deren Mischungen produzieren und die sich zur technischen Gewinnung der Produkte eignen. Verwiesen sei beispielsweise auf die folgenden deutschen Offenlegungs- oder Patentschriften: DE 19 40 488, DE 20 18 451, DE 20 44 161, DE 21 01 803, DE 21 21 397 und DE 29 25 427 sowie auf GB 1 263 765, US 3 623 957, US 4 264 738 und EP-A 6 638. Bei den genannten Anmeldungen bzw. Patenten handelt es sich um klassische Mutationsverfahren, bei denen durch die häufige Wiederholung von Mutations- und Selektionsschritten letztendlich Produktionsstämme gewonnen werden, die im Hinblick auf Ausbeute oder Produktqualität optimiert sind. Es ist dem Fachmann bekannt, daß ein deratiger Mutationsprozeß statistisch abläuft, so daß kaum zu erwarten ist, daß dabei maßgeschneiderte Stämme entstehen, die das objektiv höchstmögliche Leistungsniveau aufweisen. Darüberhinaus können derartige Zuchtstämme auch zu Rückmutationen neigen, d.h., sie verlieren ihre günstigen Eigenschaften, zumindest teilweise und 'verwildern'.

Einen wesentlichen Fortschritt hat die Genrekombinationstechnik gebracht (Cohen und Boyer, US 4 237 224). Danach ist es möglich ein Fremdgen in einem Mikroorganismus zur Expression und zur Vermehrung zu bringen, indem man in geeigneter Weise ein Plasmid öffnet, mit der fremden doppelsträngigen Desoxyribonukleinsäure verbindet, erneut zum Ring schließt und in einen geeigneten Wirtsorganismus einführt. In Anwendung dieser Grundmethode beschreibt die europäische Patentanmeldung 0 130 756 die Isolierung von doppelsträngigen Desoxyribonukleinsäuren, die zum einen für Subtilisin BPN' und zum anderen für neutrale Proteasen (Metallo-Proteasen) codieren. Weiterhin beschrieben wird die Einfügung der doppelsträngigen Desoxyribonukleinsäuren in Vektoren und auch ihre Abwandlung. Wenngleich die gentechnologische Erzeugung und Abwandlung von Hydrolasen (i.e. Proteasen, andere Amydasen, Esterasen u.s.w.) beansprucht wird, so erscheint es doch nicht möglich, die am speziellen Beispiel Subtilisin BPN' aufgezeigten Schritte ohne erfinderisches Zutun auf andere Systeme zu übertragen. Dies gilt umsomehr als nach der Lehre der genannten europäischen Patentanmeldungen die Abtrennung des für die Hydrolase codierenden Gens aus dem Genpool des Ausgangsorganismus über eine markierte Oligonukleotidkette erfolgen soll, wobei diese Oligonukleotidkette einer Teilsequenz der Hydrolase entspricht und in ihren durch den genetischen Code vorgegebenen Variationsmöglichkeiten eingesetzt wird. Für den Fachmann ist hier ein sehr hoher experimenteller Aufwand erforderlich, d.h., es müssen auf synthetischem Wege eine Vielzahl von Oligonukleotidketten in entsprechender Reinheit synthetisiert werden. Darüber hinaus setzt die Arbeitsmethode die Kenntnis der Aminosäuresequenz des gewünschten Produktes voraus.

Die Erfinder haben sich die Aufgabe gestellt, Mikroorganismenstämme zu schaffen, die in der Lage sind, Subtilisin Carlsberg zu produzieren, wobei unter dem Oberbegriff Subtilisin Carlsberg auch insbesondere eine Variante des Enzyms zu subsumieren ist, die 2 Abwandlungen besitzt. Eine weitere Aufgabe bestand darin, geeignete, in Mikroorganismen der Gattung Bacillus inserierbare und darin stabile Hybridplasmide zu schaffen, die für die Erzeugung und Exkretion von Subtilisin Carlsberg codieren. Schließlich sollten operable, isolierte, doppelsträngige Desoxyribonukleinsäuren geschaffen werden, die für Subtilisin Carlsberg codieren und die die zur Produktion und Ausschleusung des Enzyms nötigen Nukleotidsequenzen enthalten. Eine weitere Aufgabe der Erfindung bestand darin, bei der Selektion von Carlsberg positiven Mikroorganismen einen im Vergleich zu der in der europäischen Patentanmeldung 0 130 756 beschriebenen Isolationsmethode über vorgefertigte markierte Oligonukletidsequenzen vereinfachtes immunologisches Detektionsverfahren einzusetzen.

Gegenstand der Erfindung sind in einer ersten Ausführungsform isolierte, doppelsträngige Desoxyribonukleinsäuren, bestehend aus
- einem Strukturgen, das nur für ein Polypeptid kodiert
- Start- und Stopcodons in operabler Reihenfolge
- Ribosomenbindungsstelle
- Promotor

dadurch gekennzeichnet, daß sie den folgenden Aufbau aufweisen:
- Promotor der von der RNA-Polymerase von Mikroorganismen der Gattung Bacillus erkannt wird,
- Ribosomenbindungsstelle
- Startcodon
- Strukturgen das für Subtilisin Carlsberg der Sequenz

```
GCGCAAA  CCGTTCCTTA  CGGCATTCCT  CTCATTAAAG
CGGACAAAGT  GCAGGCTCAA  GGCTTTAAGG  GAGCGAATGT
AAAAGTAGCC  GTCCTGGATA  CAGGAATCCA  AGCTTCTCAT
CCGGACTTGA  ACGTAGTCGG  CGGAGCAAGC  TTTGTGGCTG
GCGAAGCTTA  TAACAGCGAC  GGCAACGGAC  ACGGCACACA
TGTTGCCGGT  ACAGTAGCTG  CGCTTGACAA  TACAACGGGT
GTATTAGGCG  TTGCGCCAAG  CGTATCCTTG  TACGCGGTTA
AAGTACTGAA  TTCAAGCGGA  AGCGGATCAT  ACAGCGGCAT
TGTAAGCGGA  ATCGAGTGGG  CGACAACAAA  CGGCATGGAT
GTTATCAATA  TGAGCCTTGG  GGGAGCATCA  GGCTCGACAG
CGATGAAACA  GGCAGTCGAC  AATGCATATG  CAAGAGGGGT
TGTCGTTGTA  GCTGCAGCAG  GGAACAGCGG  ATCTTCAGGA
AACACGAATA  CAATTGGCTA  TCCTGGGAAA  TACGATTCTG
TCATCGCTGT  TGGTGGCGTA  GACTCTAACA  GCAACAGAGC
TTCATTTTCC  AGCGTCGGAG  CAGAGCTTGA  AGTCATGGCT
CCTGGCGCAG  GCGTATACAG  CACTTACCCA  ACGAACACTT
ATGCAACATT  GAACGGAACG  TCAATGGCTT  CTCCTCATGT
AGCGGCAGCA  GCAGCTTTGA  TCTTGTCAAA  ACATCCGAAC
CTTTCAGCTT  CACAAGTCCG  CAACCGTCTC  TCCAGCACGG
CGACTTATTT  GGGAAGCTCC  TTCTACTATG  GGAAGGTCT
GATCAATGTC  GAAGCTGCCG  CTCAA
```

oder dessen Teilstücke mit proteolytischer Aktivität oder Varianten mit proteolytischer Aktivität einschließlich deren Leader-Sequenzen codiert
- Stopcodon,
wobei an beiden Enden DNA-Sequenzen mit bis zu 400 Basenpaaren ohne spezifische Expressionsfähigkeit vorhanden sein können.

Die erfindungsgemäßen isolierten doppelsträngigen Desoxyribonukleinsäuren enthalten die einzelnen Teilsequenzen in operabler Reihenfolge, d.h., daß sie in funktioneller Beziehung zu einander stehen. Darunter ist zu verstehen, daß ein Promotor vorhanden ist, der die Erkennungssequenz für die RNA-Polymerase besitzt und das diesem eine Bindungsstelle für die Ribosomen folgt. Es folgt dann ein in Bacillus operables Startcodon, vorzugsweise das Starttriplet GTG. Dem Starttriplet folgt dann die eigentliche Signalsequenz für das Leaderprotein und anschließend für das maturierte Subtilisin Carlsberg oder dessen Varianten bzw. Teilstücke. Unter Leadersequenz wird hier eine Prosequenz, Presequenz oder eine Pre/-Prosequenz verstanden, die vorzugsweise für die Ausschleusung des Enzyms aus der Zelle verantwortlich ist. Die Struktursequenz ist schließlich durch ein Stopcodon begrenzt, vorzugsweise durch ein von Bacillus erkanntes Stoptriplet, wie beispielsweise TAA. Danach kann sich eine Terminatorsequenz anschließen, wobei Sequenzen bevorzugt sind, die sich in sich selber paaren können und damit zur Schleifenbildung führen (Stem-loop). Derartige Terminatorsequenzen haben die Funktion, die Synthese der messenger-RNA für das betreffende Genprodukt zu begrenzen.

An beiden Enden der geschilderten isolierten, doppelsträngigen Desoxyribonukleinsäuren können sich weitere Sequenzen anschließen, vorzugsweise solche, denen keine spezielle Expressionswirkung zugeschrieben werden kann.

Die Sequenz für das maturierte Enzym kann im Rahmen der Erfindung gewissen Variationen unterworfen sein. Nach einer ersten Ausführungsform werden Sequenzen bereitgestellt, die für Subtilisin Carlsberg kodieren. Bevorzugt sind Sequenzen, die nur aus Codons aufgebaut sind, die in Bacillus gebräuchlich sind. Nach einer bevorzugten Ausführungsform der Erfindung werden isolierte Desoxyribonukleinsäuren beansprucht, die die folgende Sequenz für das maturierte Enzym aufweisen:

```
          10           20           30           40

....GCGCAAA  CCGTTCCTTA  CGGCATTCCT  CTCATTAAAG
CGGACAAAGT  GCAGGCTCAA  GGCTTTAAGG  GAGCGAATGT
AAAAGTAGCC  GTCCTGGATA  CAGGAATCCA  AGCTTCTCAT
CCGGACTTGA  ACGTAGTCGG  CGGAGCAAGC  TTTGTGGCTG
GCGAAGCTTA  TAACACCGAC  GGCAACGGAC.ACGGCACACA
TGTTGCCGGT  ACAGTAGCTG  CGCTTGACAA  TACAACGGGT
GTATTAGGCG  TTGCGCCAAG  CGTATCCTTG  TACGCGGTTA
AAGTACTGAA  TTCAAGCGGA  AGCGGATCAT  ACAGCGGCAT
TGTAAGCGGA  ATCGAGTGGG  CGACAACAAA  CGGCATGGAT
GTTATCAATA  TGAGCCTTGG  GGGAGCATCA  GGCTCGACAG
CGATGAAACA  GGCAGTCGAC  AATGCATATG  CAAGAGGGGT
TGTCGTTGTA  GCTGCAGCAG  GGAACAGCGG  ATCTTCAGGA
AACACGAATA  CAATTGGCTA  TCCTGCGAAA  TACGATTCTG
TCATCGCTGT  TGGTGCGGTA  GACTCTAACA  GCAACAGAGC
TTCATTTTCC  AGCGTCGGAG  CAGAGCTTGA  AGTCATGGCT
CCTGGCGCAG  GCGTATACAG  CACTTACCCA  ACGAACACTT
ATGCAACATT  GAACGGAACG  TCAATGGCTT  CTCCTCATGT
AGCGGGAGCA  GCAGCTTTGA  TCTTGTCAAA  ACATCCGAAC
CTTTCAGCTT  CACAAGTCCG  CAACCGTCTC  TCCAGCACGG
```

CGACTTATTT GGGAAGCTCC TTCTACTATG GGAAAGGTCT

Stop

GATCAATGTC GAAGCTGCCG CTCAA  TAA....

Diese Sequenz kodiert für ein Subtilisin Carlsberg, das in der Aminosäuresequenz in den Positionen 158 und 161 abweicht.

Nach einer weiteren Ausführungsform der Erfindung werden isolierte, doppelsträngige Desoxyribonukleinsäuren beansprucht, die für nicht modifiziertes Subtilisin Carlsberg kodieren, d.h., die anstelle der Aminosäure Serin die Aminosäure Asparagin in Position 161 aufweisen, bzw. anstelle der Aminosäure Asparagin in Position 158 die Aminosäure Serin aufweisen. Daraus ergibt sich, daß die in Bacillus gebräuchlichen Basentripletts (genetischen Codes) für diese Aminosäuren in den entsprechenden isolierten Desoxyribonukleinsäuren vorhanden sein müssen.

Nach einer weiteren bevorzugten Ausführungform der Erfindung erhalten die isolierten, doppelsträngigen Desoxyribonukleinsäuren zwischen dem Starttriplett und der für das maturierte Enzym kodierenden Sequenz eine Sequenz für ein Leaderprotein.

Ein weiterer Gegenstand der Erfindung sind Hybridplasmide für Mikroorganismen der Gattung Bacillus, dadurch gekennzeichnet, daß sie in operabler Form zumindest eine doppelsträngige DNS nach Anspruch 1 enthalten. Erfindungsgemäß soll ein derartiges Hybridplasmid den folgenden Aufbau aufweisen. Sie sollen über einen Resistenzmarker verfügen und zumindest eine enzymatische Spaltstelle für spezifische Spaltenzyme aufweisen, wobei pro Spaltenzym möglichst wenig Spaltstellen, d.h. nicht mehr als 4 und vorzugsweise nicht mehr als eine Spaltstelle vorhanden ist. D.h., die Hybridplasmide sollen durch Einsatz spezieller Enzyme linearisiert werden können.

Die Funktion des Resistenzmarkers ist darin zu sehen, daß hierdurch transformierte Mikroorganismen, die die Hybridplasmide enthalten, über ihre Resistenz von solchen unterschieden werden können, die diese Hybridplasmide nicht enthalten. Dem auf diesem Gebiet tätigen Fachmann stehen eine Vielzahl von Ausgangsplasmiden zur Verfügung. Diese verfügen über Resistenzmarker gegen beispielsweise Tetracyclin, Kanamycin, Chloramphenicol, oder andere Antibiotika. Unter den denkbaren Ausgangsplasmiden zur Herstellung der Hybridplasmide sollen solche ausgewählt werden, die für Mikroorganismen der Gattung Bacillus geeignet sind. Die erfindungsgemäßen Hybridplasmide werden daher zweckmäßigerweise aus Ausgangsplasmiden gewonnen, die ihrerseits für Bacillus geeignet sind. In der Fachliteratur ist eine Vielzahl derartiger Ausgangsplasmide beschrieben worden. Eine große Zahl geeigneter Plasmide kann bei Hinterlegungsstellen, so beispielsweise bei dem Bacillus Genetic Stock Center angefordert werden. Geeignete Ausgangsplasmide sind beispielsweise die folgenden: pBCE16, pC194, pUB110, pE194, pSA2100, pPL608, pBD64. Hinweise auf diese Plasmide finden sich in der Fachliteratur an den folgenden Stellen:

Bernhard K., Schrempf H., Goebel W.,

1978, J. Bacteriol. 133: 897 - 903

Gryczan, T.J., Contente, S., and Dubnau, D.

1978, J. Bacteriol. 134: 318 - 329

Williams, D.M., Duvall, E.J. and Lovett, P.S.,

1981, J. Bacteriol. 146: 1162 - 1165

Gryczan, T., Shivakumar, A.G., and Dubnau, D.,

1980, J. Bacteriol 141: 246 - 253

Ehrlich, S.D., Bursztyn-Pettegrew. H., Stroynowski, J., and Leaderberg. J., 1977 In "Recombinat Molecules: Impact on Science and Society, S. 69 -80, Raven Press, New York

Weisblum, B. Graham, M.Y., Gryczan, T., and Dubnau, D.

1979, J. Bacteriol. 137: 635 - 643

Bei der Auswahl geeigneter Ausgangsplasmide ist darauf zu achten, daß sie durch spezifische Spaltenzyme linearisierbar sind, ohne dabei ihre charakteristischen Eigenschaften zu verlieren. Unter charakteristischen Eigenschaften sind hier die Replikationsfähigkeit, die Resistenz und die Möglichkeit zur Expression in Bacillus zu verstehen. Gängige spezifische Spaltenzyme, mit denen geeignete Ausgangsplasmide linearisiert werden können, sind beispielsweise Bam HI, Eco RI, Hin dlll und Pst I.

Die erfindungsgemäßen Hybridplasmide enthalten die doppelsträngigen isolierten Desoxyribonukleinsäuren, eingeschoben in eine oder mehrere derartiger Spaltstellen. So kann beispielsweise das Ausgangsplasmid pBCE16, das im Sinne der Erfindung besonders geeignet ist, mit Hilfe von Bam HI linearisiert werden und dann nach Einfügung der erfindungsgemäßen doppelsträngigen Desoxyribonukleinsäuren wieder zum Ring geschlossen werden. Nach einer weiteren Ausführungsform der Erfindung werden außer den ringgeschlossenen Hybridplasmiden auch die korrespondierenden ringgeöffneten Formen sowie Addukte aus 2,3 oder mehreren ringgeöffneten Hybridplasmiden beansprucht.

Ein weiterer Gegenstand der Erfindung sind Mikroorganismenstämme der Gattung Bacillus, die Hybridplasmide nach den Ansprüchen 2 und folgende enthalten. Zur Erzeugung bzw. Umwandlung geeigneter Mikroorganismenstämme wird der Fachmann solche Stämme auswählen, die Hybridplasmide aufnehmen und in denen diese Hybridplasmide hinreichende Stabilität aufweisen. Bevorzugt können Stämme der Art Bacillus subtilis, Bacillus licheniformis, Bacillus amyloliquefaciens oder auch Bacillus cereus transformiert werden. Dabei können transformierte Stämme von nichttransformierten durch Detektions der gebildeten Protease z.B. auf dem nachfolgend beschriebenen immunologischen Weg unterschieden werden.

Nach einer Ausführungsform der Erfindung werden Bacillus Stämme, beispielsweise Bacillus subtilis Stämme transformiert, die ihrerseits nicht in der Lage sind, Protease zu bilden. Derartige Stämme sind von Hinterlegungsstellen beziehbar. Es gelingt so, Stämme zu erhalten, die nur das gewünschte Subtilisin Carlsberg oder die beschriebene Variante von Subtilisin Carlsberg herstellen können. In ähnlicher Weise können jedoch auch Stämme erzeugt werden, die zusätzlich zu anderen Proteasen oder Amylasen dann Subtilisin Carlsberg ausscheiden. Namentlich ist es möglich, auf diesem Wege durch Selektion Stämme mit besonders hoher Enzymproduktion zu gewinnen.

Zur Isolierung der erfindungsgemäßen doppelsträngigen Desoxyribonukleinsäuren geht man von proteasebildenden Stämmen der Art Bacillus licheniformis aus. Geeignet sind hier beispielsweise die folgenden Stämme: Bacillus licheniformis: ATCC 10716, DSM 641 sowie die Stämme DSM 3406 und DSM 3407 (Laborbezeichnung "A 441" und "A 453"). Diese werden gezüchtet wie in der deutschen Patentschrift 29 25 427 beschrieben. Danach werden die Zellen geerntet. Die geernteten Zellen werden der Protoplastierung unterworfen, d.h., es wird mit geeigneten Enzymen insbesondere Lysozym, das Murein-Gerüst aufgelöst und sodann mit Detergentien wie beispielsweise Natriumdodecylsulfat, die eigentliche Zellmembran aufgelöst. Die so entstandenen Lysate können zunächst einer Behandlung mit RNA-spaltenden Enzymen (RNase) oder/und Proteinase K unterzogen werden. Danach folgt der Isolierungsschritt der DNA.

Zur Isolierung der chromosomalen DNA sind dem Fachmann eine Reihe von Methoden bekannt, die entweder einzeln oder kombiniert durchgeführt werden können. Nach einer wichtigen Methode wird das Lysat zunächst einer fraktionierten Fällung in Ethanol unterzogen, wobei hochmoleklulare DNA-Stücke abgetrennt und somit isoliert werden können. Eine weitere Methode ist die Auftrennung in einem Dichtegradienten. Dazu wird in einer Cäsiumchlorid/DNA-Lösung ein Dichtegradient erzeugt. Die Gesamtmenge wird in verschiedene Fraktionen unterteilt, so daß sich die DNA in einzelnen dieser Fraktionen ansammelt, die dann abgetrennt und weiter verarbeitet werden können. In vielen Fällen ist es zweckmäßig, die Hauptmenge an Proteinen vor oder während der DNA-Isolierung durch Extraktion mit Chloroform/Isoamylalkohol-Gemisch zu beseitigen.

Die Abtrennung der Plasmid-DNA kann grundsätzlich in gleicher Weise geschehen, jedoch ist es dabei zweckmäßig im Cäsiumchlorid Dichtegradienten Ethidiumbromid (roter Farbstoff, der zur Intercalation befähigt ist) einzuführen und damit künstlich einen Dichteunterschied zwischen Plasmid und Chromosomal-DNA herbeizuführen.

Die gereinigten isolierten Desoxyribonukleinsäuren werden sodann in geeignetem Puffer (bei pH-Werten zwischen 7 und 7,5) mittels Restriktionsenzymen gespalten. Geeignete Restriktionsenzyme sind beispielsweise Bam HI, Sau 3A oder andere Enzyme, die DNA an spezifischen Stellen zu spalten vermögen. Dabei kann die chromosomale DNA mit den gleichen oder auch unterschiedlichen Enzymen wie die Plasmid DNA behandelt werden. In jedem Falle hat der Fachmann darauf zu achten, daß die Spaltung der chromosomalen DNA nur unvollständig durchgeführt wird, um so Spaltstücke ausreichender Länge zu erhalten, die mit hinreichender Wahrscheinlichkeit das Strukturgen in nichtzerstörter Weise enthalten. Die isolierten doppelsträngigen Desoxyribonukleinsäuren werden mit den linearen Plasmiden vermischt. Dabei werden vorzugsweise die chromosomalen Desoxyribonukleinsäuren in einem hohen Gewichtsüberschuß gegenüber den linearisierten Plasmiden eingesetzt. Vorzugsweise wird ein Verhältnis von 1 : 2 bis 1 : 20, insbesondere von 1 : 5 bis 1 : 10 eingesetzt. Die gemischten Desoxyribonukleinsäuren, die dann bei einer Konzentration von etwa 0,2 mg pro ml vorliegen sollen, werden in einem darauffolgenden Schritt mittels DNA-Ligase verknüpft.

Mit dem so erhaltenen Ligationsgemisch werden kompetente Zellen von Bacillus-Arten wie Bacillus subtilis oder Bacillus licheniformis transformiert. Will man erfolgreich transformierte Stämme an ihrer Proteasebildung erkennen, so ist es bevorzugt, solche Stämme zu transformieren, die ihrerseits nicht in der Lage sind, Protease zu bilden oder wenigstens nicht in der Lage sind Subtilisin Carlsberg zu bilden. Es können jedoch auch die Ausgangsstämme transformiert werden. Um kompetente Zellen zu erhalten, wird der zur Transformation vorgesehene Stamm in einem Minimalmedium bis zur stationären Phase angezogen und danach in einem verdünnten zweiten Minimalmedium weitergezogen. Den so vorbehandelten Mikroorganismen wird meist in Pufferlösung dann das Hybridplasmid als solches angeboten. Danach kann sich eine Wachstungsphase in einem Vollmedium anschließen (Literatur Cahn, F.H. and Fox, M.S. (1968) J. Bacteriol. 95 : 867 - 875). Die so erhaltenen Mikroorganismen werden sodann einem Selektionsverfahren unterworfen. Dabei wird üblicherweise zunächst in einem Medium angezüchtet, das ein Antibiotikum enthält, gegen das das Hybridplasmid Resistenz vermittelt. Auf diese Weise können in einem ersten Selektionsschritt Organismen erhalten werden, die tatsächlich das Hybridplasmid in einer oder mehreren Kopien enthalten. Diese Mikroorganismen werden dann in einem weiteren Selektionsschritt hinsichtlich ihrer Fähigkeit, Subtilisin Carlsberg zu bilden, selektioniert.

Für diesen Selektionsschritt kann vorzugsweise eine immunologische Methode eingesetzt werden. Eine bevorzugte Methode basiert auf einem Verfahren von Broome und Gilbert. Hierzu werden zunächst, wie von Buckel beschrieben, Antikörper gegen Subtilisin Carlsberg durch Immunisierung von Kaninchen gewonnen. Ein Teil der Antikörper wird durch Ammoniumsulfatfällung und DEAE-Chromatographie, ein anderer Teil zusätzlich noch durch Affinitätschromatographie mit aktivierter Sepharose 4B angereichert.

Der Nachweis von Subtilisin Carlsberg kann wie folgt erbracht werden:

Eine PVC-Folie wird mit Antikörpern gegen Subtilisin Carlsberg beschichtet. Die beschichtete Folie wird auf die zu untersuchenden Bakterienkulturen gelegt. Dabei wird vorhandenes Subtilisin Carlsberg von den Antikörpern gebunden. In einem weiteren Schritt werden an den Antikörper-Subtilisin-Carlsberg-Komplex die affinitätschromatographisch gereinigten Subtilisin Carlsberg Antikörper gebunden, an die in einem vorherigen Schritt Peroxydase gekoppelt worden war.

(1) Broome, S. & Gilbert, W. (1978) Proc. Natl. Acad. Sci. USA 75, 2746 - 2749

(2) Buckel, P & Zehelein, E. (1981) Gene 16, 149 - 159

Die Detektion wird mit Tetramethylbenzidin als Peroxydase-Substrat durchgeführt. Bei Anwesenheit von Subtilisin Carlsberg positiven Klonen entwickelt sich eine blaugrüne Farbe.

Die geschilderte immunologische Trennmethode bietet im Zusammenhang mit der Erfindung große Vorteile. So wird es dadurch möglich, gezielt solche Stämme zu erkennen, die tatsächlich Subtilisin Carlsberg bilden. Dies ist besonders von Bedeutung, wenn mit Stämmen gearbeitet wird, die auch noch andere nicht auf das immunologische Verfahren ansprechende Stoffwechselprodukte aufweisen. Ein weiterer Vorteil des Detektionsverfahrens liegt darin, daß diese Arbeitsweise es ermöglicht, aus einer großen Vielzahl transformierter und nicht transformierter Stämme die Klone mit Sicherheit auswählen zu können.

Aus den so isolierten Klonen, die die erfindungsgemäßen Hybridplasmide enthalten, können die Hybridplasmide in der Anfangs beschriebenen Art und Weise durch Zelllyse und anschließend Abtrennung im Dichtegradienten in Gegenwart von Ethidiumbromid isoliert werden. Auf diese Weise stehen Hybridplasmide in gereinigter Form zur Verfügung. Störendes Ethidiumbromid kann dabei durch Extraktion mit Isopropanol abgetrennt werden. Die gereinigten Hybridplasmide lassen sich in andere Bacillus-Stämme unter den oben beschriebenen Bedingungen einführen.

Nach einer weiteren Ausführungsform der Erfindung kann die Transformation anstatt über kompetente Zellen über Protoplasten durchgeführt werden. Dazu werden zunächst Protoplasten hergestellt, indem man die Murein-Zellwand von Mikroorganismen der Gattung Bacillus mit geeigneten Enzymen auflöst und danach die Hybridplasmide zusammen mit Polyethylenglykol auf die Protoplasten einwirken läßt. Dazu wird vorzugsweise in Pufferlösungen gearbeitet. Die Protoplasten werden dann auf einen Regenerierungsmedium weiter gezüchtet und die so erhaltenen intakten Mikroorganismen, die das erfindungsgemäße Hybridplasmid enthalten, nach den vorgenannten Selektionsverfahren von anderen Stämmen abgetrennt.

Chromosomale DNA aus Bacillus licheniformis DSM 641 oder auch ATCC 10716 wurde nach der Methode von Marmur (1) isoliert, mit den folgenden Abwandlungen: Die Zellen wurden in 1 mg Lysozym pro ml in 10 mM Tris-hydrochlorid pH 7,5 mit 25 % Saccharose 20 Minuten in Eis inkubiert bevor EDTA und Natriumdodecylsulfat zugegeben wurde. Die Extraktion mit Chloroform-Isoamylalkohol wurde nur einmal durchgeführt.

Die DNA wurde dann 20 Stunden mit 42.000 UpM bei 20°C in einem Dichtegradienten (Dichte 1,71 g/cm$^3$) mit Hilfe eines Vertikalrotors zentrifugiert und von anderen Zellbestandteilen abgetrennt und durch Austropfen fraktioniert.

Plasmid-DNA aus Bacillus subtilis wurde nach der Methode von Bernhard (1) isoliert, wobei die Plasmid-DNA nach der Dichtegradientenzentrifugation mittels einer Kanüle abgezogen wurde.

Als Vektorplasmid für die Klonierung in Bacillus subtilis BR 151 (3) wurde pBCE 16 (2, 4) verwendet.

Um das Gen für Subtilisin Carlsberg intakt zu erhalten, wurde eine partielle Spaltung der chromosomalen DNA von Bacillus licheniformis DSM 641 oder ATCC 10716 mit der Restriktionsendonuclease Sau 3A durchgeführt.

Da Sau 3A eine 4er Sequenz erkennt, Bam HI jedoch eine 6er-Sequenz, Sau 3A also öfter schneidet, sind die Spaltstellen für eine unvollständige Sau 3A-Spaltung gleichmäßiger verteilt als die für eine Spaltung der chromosomalen DNA mit Bam HI.

Die Spaltung wurde wie in allen folgenden Fällen entsprechend den Bedingungen des Handbuchs von Maniatis (5) durchgeführt, jedoch für die unvollständige Spaltung die Inkubationszeit, die sonst bei einer Stunde lag, entsprechend verkürzt, um Spaltprodukte zu erhalten, die schwerpunktmäßig in der Größe des Vektors pBCE 16 (ca. 3 Md) liegen. Pro 1 μg DNA wurde ca. 1 Unit Enzym eingesetzt. Der Vektor pBCE 16 wurde mit der Restriktionsendonuclease Bam HI linearisiert, das die gleichen überlappenden Enden wie Sau 3A entstehen läßt.

Nach der Inkubation wurde das Enzym zweimal mit dem halben Volumen Phenol extrahiert, dann Bam HI-gespaltenes pBCE 16 und Sau 3A-gespaltene chromosomale DNA im mengenmäßigen Verhältnis 1 : 10 gemischt, das Gemisch 5mal mit gleichem Volumen Ether extrahiert und sodann mit doppeltem Volumen Ethanol 20 Minuten bei -70°C inkubiert und die DNA durch Zentrifugation pelletiert.

Nach Trocknen wurde die DNA in folgendem Puffer gelöst: 66 mM Tris-(hydroxymethyl)-aminomethan, 5mM $MgCl_2$, 0,3 mM Adenosintriphosphat, 1,5 mM Dithiothreit und 0,07 mg/ml Rinderserumalbumin.

Die DNA-Konzentration wurde auf 200 ug/ml eingestellt und T4-DNA-Ligase in einer Konzentration von 1 U pro 1 μg eingesetzter DNA zugegeben. Die Ligasereaktion wurde ca. 18 Stunden bei 16°C durchgeführt. Das Ligationsgemisch, d.h. die mittels T4-DNA-Ligase verknüpften DNA-Fragmente wurden dann zur Transformation kompetenter Zellen von Bacillus subtilis BR 151 verwendet.

Kompetente Zellen von B. subtilis BR 151 wurden nach der Methode von Laird hergestellt, wie sie von Cahm und Fox (6) beschrieben wurde.

Zu 0,82 ml kompetenten Zellen wurden 0,18 ml 0,1 M $MgCl_2$, 0,13 ml 0,05 M $CaCl_2$ und 0,13 ml 100 mM EGTA (Ethylenglykol-bis-2-aminoethylether-N,N,N,N'-tetraacetat) pH 7,3 (in dieser Reihenfolge) zugegeben und jeweils durchmischt, nach 5 Minuten leichtem Schütteln bei 30°C dann 10 μl bzw. 2 μg des Ligationsgemisches. Nach 30 Minuten langsamen Schütteln (60 UpM) bei 30°C wurde 1 ml 2fach HGP (10 g Pepton aus Gasen, 5 g Hefeextrakt, 5 g NaCl und 5 g Glucose/pro Liter) zugegeben.

Nach 90 Minuten Schütteln mit 160 UpM bei 30°C wurden die Zellen auf Calcium-Caseinat-Agar (nach Frazier und Rupp modifiziert - Merck) mit zusätzlich 0,5 % Gasen und 15 μg/ml Tetracyclin zur Selektionierung der transformierten Zellen zu 100 ul pro Platte mit einem Glasspatel verteilt.

Die Platten wurden 48 Stunden bei 37°C bebrütet.

Mittels chromatographisch an Diethylaminoethylcellulose (DEAE) gereinigter Subtilisin Carlsberg-spezifischer Antikörper in Kombination mit einer von Boehringer/Mannheim lieferbaren "Test-Combination zum immunologischen Nachweis spezifischer Gen-Expression in Mikroorganismen" konnten 6 proteasepositive Klone unter insgesamt ca. 32.000 Transformanten nachgewiesen werden. Einer dieser Klone erwies sich nach mehrmaligem Überimpfen als ausreichend stabil für weitere Untersuchungen. Der Nachweis von S. Carlsberg wurde wie folgt durchgeführt:

PVC-Folien wurden zur Entfettung 2 Minuten in Isopropanol gebadet, getrocknet und 15 Folien in 40 ml 0,2 M Natriumcarbonatpuffer pH 9,2 mit 0,6 ml DEAE-gereinigte Subtilisin Carlsberg-spezifische IgG-Fraktion 10 Minuten zur Beschichtung inkubiert. Ohne Trocknung werden die Folien in ein Nachbeschichtungsbad überführt und 10 Minuten darin belassen:

40 ml PBS-Puffer[+]

+ 4,8 mg Rinder-Globulin

+ 40 mg Rinder-Serumalbumin

[+]) PBS-Puffer pH 7,5: 0,05 M $KH_2PO_4$; 0,1 M NaCl

Sodann wurden die Folien zwischen Papierhandtüchern getrocknet.

Die IgG beschichteten Folien wurden dann auf die bewachsenen Agarplatten aufgelegt und 2 Stunden bei Raumtemperatur inkubiert. Nach Abnehmen der Folien wurden die anhaftenden Zellen mit kaltem Leitungswasser abgespült und beidseitig mit auf 6°Ctemperiertem PBS-Puffer + 0,1 % Rinderserumalbumin abgespült. Ohne Trocknung wurden die Folien in 50 ml Konjugatbad (50 ml PBS-Puffer mit 0,1 % Rinderserumalbumin + 1 μl Peroxidase-konjugiertes IgG gegen Subtilisin Carlsberg) überführt und darin 4 Stunden bei Raumtemperatur belassen.

Die Folien wurden dann mit kaltem Leitungswasser abgespült und anschließend beidseitig mit 6°C temperiertem PBS-Puffer + 0,05 % Tween 20 + 0,1 % Rinderserumalbumin abgespült. Nach Trocknen zwischen Papierhandtüchern wurden die . Folien auf einem Substrat (Boehringer Gen-Expressionskit[R] Boehringer Mannheim Gm 611) für die gekoppelte Peroxidase inkubiert. Es handelt sich dabei um Tetramethylbenzidin, das in einer Gelantinegrundlage gelöst ist. Nach 10 bis 30 Minuten Inkubation bei Raumtemperatur entwickelt sich bei positiven Kolonien, d.h. bei Anwesenheit von Subtilisin Carlsberg, eine blaugrüne Farbe.

Die Plasmid-DNA des stabilen S. Carlsberg-positiven Klons (pC 50) wurde wie oben beschrieben isoliert.

Zur Charakterisierung der pC 50-DNA mittels verschiedener Restriktionsenzyme wurde einzeln und in Kombination mit Ava I, Bam HI, Bal I, Eco RI, Hpa II, Pst I und Sst I gespalten.

Die Analyse der in der vertikalen Agarosegelelektrophorese (7) aufgetrennten Spaltprodukte ergab folgende Plasmidkarte:

Beide das klonierte Fragment betreffenden Eco RI/Pst I-Fragmente und das sich links anschließende Eco RI-Fragment wurden in die Sequenzierungsvektoren pEMBL 8 bzw. pEMBL 9 (8) inseriert und die Sequenz nach der Kettenabbruch-Didesoxymethode (9) bestimmt.

Literatur:

1. Marmur, J. (1961) J, Mol. Biol. 3: 208-218
2. Bernhard, K., H. Schrempf and W. Goebel (1978) J. Bacteriol. 133: 897-903
3. Bacillus Genetic Stock Center Nr. 1A40
4. Bacillus Genetic Stock Center Nr. 1E9
5. Maniatis, T., E.F. Fritsch, J. Sambrook (1982), Cold Spring Harbor
6. Cahn, F.H. and Fox, M.S. (1968) J. Bacteriol. 95: 867-875
7. Meyers, J., D. Sanchez, L. Elwell and S. Falkow (1975) J.Bacteriol. 127: 1529-1537
8. Dente, L., G. Casareni and R. Cortese (1983), Nucleic Acids Research 11: 1645-1655
9. F. Sanger, S. Nicklen and A.R. Coulson (1977) Proc. Nat. Acad. Sci. USA 74: 5463-5467

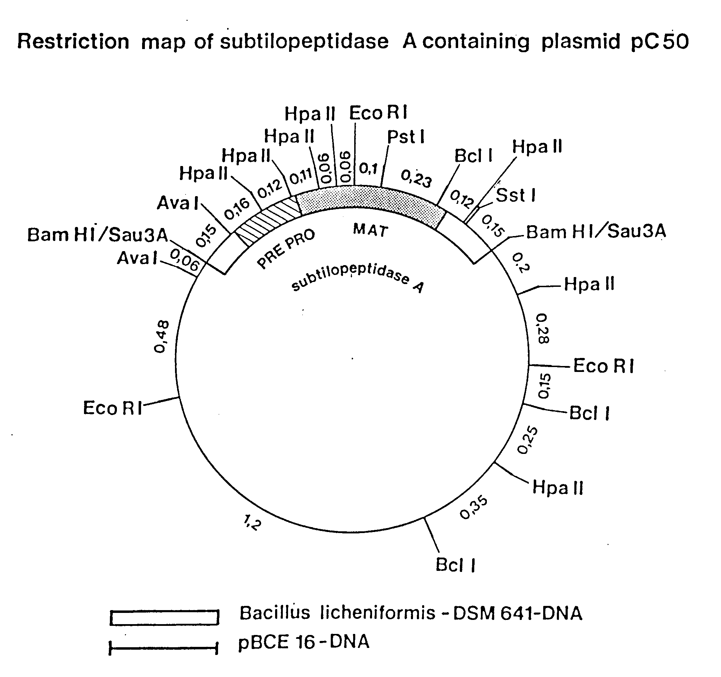

**Patentansprüche**

1.  Isolierte doppelsträngige Desoxyribonukleinsäuren, bestehend aus
    -   einem Strukturgen, das nur für ein Polypeptid kodiert
    -   Start- und Stopcodons in operabler Reihenfolge
    -   Ribosomenbindungsstelle
    -   Promoter
        dadurch gekennzeichnet, daß sie den folgenden Aufbau aufweisen:
    -   Promoter, der von der RNA-Polymerase von Mikroorganismen der Gattung Bacillus erkannt wird.
    -   Ribosomenbindungsstelle
    -   Startcodon
    -   Strukturgen das für Subtilisin Carlsberg der Sequenz

```
GCGCAAA CCGTTCCTTA CGGCATTCCT CTCATTAAAG
CGGACAAAGT GCAGGCTCAA GGCTTTAAGG GAGCGAATGT
AAAAGTAGCC GTCCTGGATA CAGGAATCCA AGCTTCTCAT
CCGGACTTGA ACGTAGTCGG CCGAGCAAGC TTTCTGGCTG
GCGAAGCTTA TAACACCGAC GGCAACGGAC ACGGCACACA
TGTTGCCGGT ACAGTAGCTG CGCTTGACAA TACAACGGGT
GTATTAGGCG TTGCGCCAAG CGTATCCTTG TACGCGGTTA
AAGTACTGAA TTCAAGCGGA AGCGGATCAT ACAGCGGCAT
TGTAAGCGGA ATCGAGTGGG CGACAACAAA CGGCATGGAT
GTTATCAATA TGAGCCTTGG GGGAGCATCA GGCTCGACAG
CGATGAAACA GGCAGTCGAC AATGCATATG CAAGAGGGGT
TGTCGTTGTA GCTGCAGCAG GGAACAGCGG ATCTTCAGGA
AACACGAATA CAATTGCCTA TCCTGCGAAA TACGATTCTG
TCATCGCTGT TGGTGCGGTA GACTCTAACA GCAACAGAGC
TTCATTTTCC AGCGTCGGAG CAGAGCTTGA AGTCATGGCT
CCTGGCGCAG GCGTATACAG CACTTACCCA ACGAACACTT
ATGCAACATT GAACGGAACG TCAATGGCTT CTCCTCATGT
AGCGGGAGCA GCAGCTTTGA TCTTGTCAAA ACATCCGAAC
CTTTCAGCTT CACAAGTCCG CAACCGTCTC TCCAGCACGG
CGACTTATTT GGGAAGCTCC TTCTACTATG GGAAAGGTCT
GATCAATGTC GAAGCTGCCG CTCAA
```

oder dessen Teilstücke mit proteolytischer Aktivität oder Varianten mit proteolytischer Aktivität einschließlich deren Leader-Sequenzen codiert
   - Stopcodon,
wobei an beiden Enden Oligonukleotidketten mit bis zu 400 Basenpaaren ohne spezifische Expressionsfähigkeit vorhanden sein können.

2. Hybridplasmide für Mikroorganismen der Gattung Bacillus, dadurch gekennzeichnet, daß sie in operabler Form zumindest eine doppelsträngige DNS nach Anspruch 1 enthalten.

3. Hybridplasmide nach dem Anspruch 2, dadurch gekennzeichnet, daß sie einen Resistenzmarker enthalten sowie für die Insertion von Fremd-DNA mindestens eine enzymspezifische Spaltstelle, wobei pro Spaltenzym vorzugsweise nicht mehr als eine Spaltstelle vorhanden ist.

4. Hybridplasmide nach Anspruch 2 und 3, dadurch gekennzeichnet, daß sie einen Resistenzmarker enthalten sowie für die Insertion von Fremd-DNA Schnittstellen für spezifische DNS spaltende Enzyme, wobei vorzugsweise für jedes Spaltenzym nicht mehr als 4 und insbesondere nur eine Spaltstelle vorhanden sind.

5. Hybridplasmide nach den Ansprüchen 2 - 4, dadurch gekennzeichnet, daß sie sich von pBCE16 ableiten und die Desoxyribonukleinsäuren nach Anspruch 1 als Einschub in die Schnittstelle BamH1 enthalten.

6. Mikroorganismenstämme der Gattung Bacillus enthaltend Hybridplasmide nach den Ansprüchen 1 - 5.

**7.** Mikroorganismenstämme nach Anspruch 6, dadurch gekennzeichnet, daß sie zu des Arten Bacillus subtilis oder Bacillus licheniformis gehören.

**8.** Verfahren zur Herstellung von Subtilisin Carlsberg, dadurch gekennzeichnet, daß man Mikroorganismenstämme der Ansprüche 6 und 7 züchtet.

**9.** Verfahren nach Anspruch 8, dadurch gekennzeichnet, daß man Mikroorganismenstämme der Ansprüche 6 und 7 züchtet, die Hybridplasmide enthalten, die für ein abgewandeltes Subtilisin Carlsberg codieren, das in der Position 158 die Aminosäure Serin anstelle der Aminosäure Asparagin aufweist und in der Position 161 Asparagin anstelle von Serin.

**Claims**

**1.** Isolated, double-stranded deoxyribonucleic acids consisting of
- a structural gene which only codes for a polypeptide
- start and stop codons in an operable sequence
- a ribosome binding site
- a promoter
characterized in that they have the following structure:
- promoter recognized by the RNA-polymerase of microorganisms of the genus bacillus
- ribosome binding site,
- start codon
- structural gene which codes for subtilisin Carlsberg of the sequence

```
GCGCAAA CCGTTCCTTA CGGCATTCCT CTCATTAAAG
CGGACAAAGT GCAGGCTCAA GGCTTTAAGG GAGCGAATGT
AAAAGTAGCC GTCCTGGATA CAGGAATCCA AGCTTCTCAT
CCGGACTTGA ACGTAGTCCG CGGAGCAAGC TTTGTCGCTG
GCGAAGCTTA TAACACCGAC GGCAACGGAC ACGGCACACA
TGTTGCCGGT ACAGTAGCTG CGCTTGACAA TACAACGGGT
GTATTAGGCG TTGCGCCAAG CGTATCCTTG TACGCGGTTA
AAGTACTCAA TTCAAGCGGA AGCGGATCAT ACAGCGGCAT
TCTAAGCGGA ATCGAGTGGG CGACAACAAA CGGCATGGAT
GTTATCAATA TGAGCCTTGG CGGAGCATCA GCCTCGACAG
CGATGAAACA GGCAGTCGAC AATGCATATG CAAGAGGGGT
TGTCGTTGTA GCTGCAGCAG GGAACAGCGG ATCTTCAGGA
AACACGAATA CAATTGCCTA TCCTGCGAAA TACGATTCTG
TCATCGCTGT TGGTGCCGTA GACTCTAACA GCAACAGAGC
TTCATTTTCC AGCGTCGGAG CAGAGCTTGA AGTCATGGCT
CCTGGCGCAG GCCTATACAG CACTTACCCA ACGAACACTT
ATGCAACATT GAACGGAACG TCAATGGCTT CTCCTCATGT
AGCGGGAGCA GCAGCTTTGA TCTTGTCAAA ACATCCGAAC
CTTTCAGCTT CACAAGTCCG CAACCGTCTC TCCAGCACGG
CGACTTATTT GGGAAGCTCC TTCTACTATG GGAAAGGTCT
GATCAATGTC GAAGCTGCCG CTCAA
```

or its proteolytically active sub-units or proteolytically active variants, including their leader sequences,
- stop codon,
DNA-sequences containing up to 400 base pairs without any special expression effect optionally being present at both ends.

2. Hybrid plasmids for microorganisms of the genus bacillus, characterized in that they contain at least one double-stranded DNA according to claim 1 in operable form.

3. Hybrid plasmids as claimed in claim 2, characterized in that they contain a resistance marker and, for the insertion of foreign DNA, at least one enzyme-specific cleavage site, preferably no more than 1 cleavage site being present per restriction enzyme.

4. Hybrid plasmids as claimed in claims 2 and 3, characterized in that they contain a resistance marker and, for the insertion of foreign DNA, cleavage sites for specific DNA-cleaving enzymes, preferably no more than four and more preferably only one cleavage site being present for each restriction enzyme.

5. Hybrid plasmids as claimed in claims 2 to 4, characterized in that they are derived from pBCE16 and the deoxyribonucleic acids according to claim 1 contain BamHI inserted into the cleavage site.

6. Microorganism strains of the genus bacillus containing hybrid plasmids according to claims 1 to 5.

7. Microorganism strains as claimed in claim 6, characterized in that they belong to the species Bacillus subtilis or Bacillus licheniformis.

8. A process for the preparation of subtilisin Carlsberg, characterized in that microorganism strains according to claims 6 and 7 are cultivated.

9. A process as claimed in claim 8, characterized in that microorganism strains according to claims 6 and 7 are cultivated which contain hybrid plasmids coding for a modified subtilisin Carlsberg which contains the amino acid serine instead of the amino acid apsaragine in position 158 and asparagine instead of serine in position 161.


**Revendications**

1. Acides désoxyribonucléiques bicaténaires isolés, constitués de :
   - un gène structurel qui code pour un polypeptide
   - des codons d'initiation et d'arrêt en séquence fonctionnelle,
   - site de fixation ribosomique
   - promoteur,
   caractérisés en ce qu'ils présentent la structure suivante :
   - promoteur, qui est reconnu par l'ARN-polymérase de micro-organismes appartenant au type bacillus,
   - site de fixation ribosomique
   - codon d'initiation
   - gène structural qui code pour la subtilisine Carlsberg de séquence

```
       GCGCAAA    CCGTTCCTTA  CGGCATTCCT  CTCATTAAAG
    CGGACAAAGT GCAGGCTCAA GGCTTTAAGG GAGCGAATGT AAAAGTAGCC
    GTCCTGCATA CAGGAATCCA ACCTTCTCAT CCGGACTTGA ACGTAGTCGG
    CGGAGCAAGC TTTGTGGCTG GCGAAGCTTA TAACACCGAC GGCAACGGAC
    ACGGCACACA TGTTGCCGGT ACAGTAGCTG CGCTTGACAA TACAACGGGT
    GTATTAGGCG TTGCGCCAAG CGTATCCTTG TACGCGGTTA AAGTACTGAA
    TTCAAGCGGA AGCGGATCAT ACAGCCGCAT TGTAAGCGGA ATCGAGTGGG
    CGACAACAAA CGGCATGGAT GTTATCAATA TGAGCCTTGG GGGAGCATCA
    GGCTCGACAG CGATGAAACA GGCAGTCGAC AATGCATATG CAAGAGGGGT
    TGTCGTTGTA GCTGCAGCAG GGAACAGCGG ATCTTCAGGA AACACGAATA
    CAATTGGCTA TCCTGCGAAA TACGATTCTG TCATCGCTGT TGGTGCGGTA
    GACTCTAACA GCAACAGAGC TTCATTTTCC AGCGTCGGAG CAGAGCTTGA
    AGTCATGGCT CCTGGCGCAG GCGTATACAG CACTTACCCA ACGAACACTT
    ATGCAACATT GAACGGAACG TCAATGGCTT CTCCTCATGT AGCGGGAGCA
    GCAGCTTTGA TCTTGTCAAA ACATCCGAAC CTTTCAGCTT CACAAGTCCG
    CAACCGTCTC TCCAGCACGG CGACTTATTT GGGAAGCTCC TTCTACTATG
    GGAAAGGTCT GATCAATGTC GAAGCTGCCG CTCAA
```

ou des fragments de celle-ci à activité protéolytique ou des variants à activité protéolytique, y compris leurs séquences leader
- codon d'arrêt
des chaînes d'oligonucléotides comportant jusqu'à 400 paires de bases sans capacité d'expression spécifique pouvant être présentes aux deux extrémités.

2. Plasmides hybrides pour micro-organismes appartenant au genre Bacillus, caractérisés en ce qu'ils contiennent, sous forme fonctionnelle, au moins un ADN bicaténaire selon la revendication 1.

3. Plasmides hybrides selon la revendication 2, caractérisés en ce qu'ils contiennent un marqueur de résistance ainsi que, pour l'insertion d'ADN étranger, au moins un site de coupure spécifique d'une enzyme, de préférence pas plus d'un site de coupure n'étant présent par enzyme de coupure .

4. Plasmides hybrides selon la revendication 2 ou 3, caractérisés en ce qu'ils contiennent un marqueur de résistance ainsi que, pour l'insertion d'ADN étranger, des sites de coupure pour des enzymes spécifiques coupant l'ADN, de préférence pas plus de 4 sites de coupure n'étant présents et, en particulier, seul un site de coupure étant présent pour chaque enzyme de coupure .

5. Plasmides hybrides selon l'une quelconque des revendications 2 à 4, caractérisés en ce qu'ils dérivent de pBCE 16 et contiennent les acides désoxyribonucléiques selon la revendication 1, en tant qu'élément d'insertion, dans le site de coupure BamH1.

6. Souches de micro-organismes appartenant au genre Bacillus contenant des plasmides hybrides selon les revendications 1 à 5.

7. Souches de micro-organismes selon la revendication 6, caractérisées en ce qu'elles appartiennent aux espèces Bacillus subtilis ou Bacillus licheniformis.

8. Procédé pour la production de subtilisine Carlsberg, caractérisé en ce que l'on cultive des souches de micro-organismes selon les revendications 6 et 7.

9. Procédé selon la revendication 8, caractérisé en ce que l'on cultive des souches de microorganismes selon les revendications 6 et 7, qui contiennent des plasmides hybrides qui codent pour une subtilisine Carlsberg modifiée et comporte en position 158, l'aminoacide sérine au lieu de l'aminoacide aspragine, et en position 161 l'asparagine au lieu de la sérine.

# Restriction map of subtilopeptidase A containing plasmid pC50

□ Bacillus licheniformis - DSM 641-DNA

├─────┤ pBCE 16-DNA